(19) Europäisches Patentamt
European Patent Office
Office européen des brevets

(11) Veröffentlichungsnummer: **0 162 346**
**B1**

(12) # EUROPÄISCHE PATENTSCHRIFT

(45) Veröffentlichungstag der Patentschrift: **03.06.87**

(51) Int. Cl.[4]: **C 07 C 119/048**

(21) Anmeldenummer: **85105232.4**

(22) Anmeldetag: **30.04.85**

(54) **Neue Triisocyanate, ein Verfahren zu ihrer Herstellung und ihre Verwendung zur Herstellung von Polyurethankunststoffen.**

(30) Priorität: **12.05.84 DE 3417684**

(43) Veröffentlichungstag der Anmeldung: **27.11.85 Patentblatt 85/48**

(45) Bekanntmachung des Hinweises auf die Patenterteilung: **03.06.87 Patentblatt 87/23**

(84) Benannte Vertragsstaaten: **DE FR GB IT NL**

(56) Entgegenhaltungen:
**EP-A-0 046 917**
**EP-A-0 113 043**
**EP-A-0 113 044**
**FR-A-1 447 964**
**US-A-2 729 666**
**US-A-3 663 514**

(73) Patentinhaber: **BAYER AG, Konzernverwaltung RP Patentabteilung, D-5090 Leverkusen 1 Bayerwerk (DE)**

(72) Erfinder: **Knöfel, Hartmut, Dr., Dülmener Weg 21, D-5068 Odenthal (DE)**
Erfinder: **Penninger, Stefan, Dr., Hahnenstrasse 106, D-5024 Pulheim (DE)**
Erfinder: **Brockelt, Michael, Neuenhauser Weg 1, D-5060 Bergisch-Gladbach 2 (DE)**
Erfinder: **Hammen, Günter, Dr., Bunzlauer Weg 13, D-4049 Rommerskirchen 1 (DE)**
Erfinder: **Stutz, Herbert, Dr., Schulstrasse 81, D-4047 Dormagen 5 (DE)**

**EP 0 162 346 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die Erfindung betrifft neue Triisocyanate, die sowohl aromatisch als auch cycloaliphatisch gebundene Isocyanatgruppen aufweisen, und die wertvolle Aufbaukomponenten mit abgestufter Reaktivität der Isocyanatgruppen bei der Herstellung von Polyurethankunststoffen darstellen, ein Verfahren zu ihrer Herstellung durch Phosgenierung der ihnen zugrundeliegenden Triamine sowie ihre Verwendung als Aufbaukomponente bei der Herstellung von Polyurethankunststoffen, insbesondere bei der Herstellung von Polyurethanlacküberzügen nach dem Isocyanat-Polyadditionsverfahren.

Araliphatische Polyisocyanate wie z.B. das Isocyanatgruppen aufweisende Mischtrimerisat von Hexamethylendiisocyanat und Diisocyanatotoluol eignen sich in hervorragender Weise zur Herstellung von Polyurethankunststoffen, insbesondere von Polyurethanlacküberzügen, da sich die positiven Eigenschaften von aromatischen Isocyanaten mit denen von aliphatischen Isocyanaten in günstiger Weise ergänzen. Polyurethan-Lacke auf Basis von derartigen araliphatischen Lackpolyisocyanaten zeichnen sich durch folgende vorteilhaften Eigenschaften aus (vgl. H. Wagner, H.F. Sarx, «Lackkunstharze», Carl Hanser Verlag, München, 5. Auflage 1971, Seiten 163-164):

- Rasche Trocknung
- Erhöhte Vergilbungsresistenz der Lacküberzüge im Vergleich zu solchen auf Basis von aromatischen Polyisocyanaten
- Erhöhte Reaktivität und damit kürzere Trocknungszeiten im Vergleich zu entsprechenden Lacken auf Basis von aliphatischen Polyisocyanaten
- Gute Farbtonbeständigkeit der pigmentierten Lacke
- Verbesserte Glanzhaltung und Wetterbeständigkeit im Vergleich zu entsprechenden Lacken auf Basis von aromatischen Polyisocyanaten
- Relativ lange Topfzeit der Lacke.

Die Herstellung derartiger araliphatischer Polyisocyanate (vgl. z.B. DE-PS 1 670 667) ist allerdings recht aufwendig, da u.a. die nach der Trimerisierung vorliegenden überschüssigen Ausgangsdiisocyanate in einem zusätzlichen Verfahrensschritt abgetrennt werden müssen. Nachteilhaft ist ferner, dass durch die Trimerisierungsreaktion wertvolle Isocyanatgruppen verbraucht werden und auch, dass die Mischtrimerisate nach ihrer Herstellung nicht destillativ gereinigt werden können.

Es war die der Erfindung zugrundeliegende Aufgabe, neue Polyisocyanate zur Verfügung zu stellen, die einerseits die Vorteile der genannten Lackpolyisocyanate des Standes der Technik mit Isocyanatgruppen unterschiedlicher Reaktivität aufweisen, und die andererseits nicht mit den genannten Nachteilen behaftet sind.

Diese Aufgabe konnte durch die Bereitstellung der nachstehend näher beschriebenen erfindungsgemässen Polyisocyanate und des zu ihrer Herstellung geeigneten Verfahrens gelöst werden.

Gegenstand der Erfindung sind neue, gegebenenfalls Stellungs- und/oder Stereoisomerengemische darstellende Triisocyanate der Formel (I)

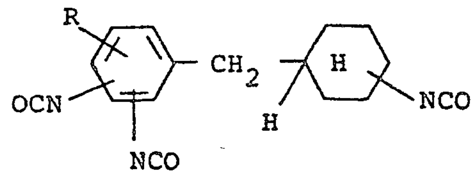
(I)

in welcher

R für Wasserstoff oder einen verzweigten oder linearen Alkylrest mit 1 bis 4 Kohlenstoffatomen, vorzugsweise für Wasserstoff oder eine Methylgruppe steht.

Gegenstand der Erfindung ist auch ein Verfahren zur Herstellung dieser neuen Triisocyanate, welches dadurch gekennzeichnet ist, dass man die ihnen zugrundeliegenden Polyamine phosgeniert.

Gegenstand der Erfindung ist schliesslich auch die Verwendung der neuen Triisocyanate als Aufbaukomponente von Polyurethankunststoffen nach dem Isocyanat-Polyadditionsverfahren.

Ausgangsmaterialien für das erfindungsgemässe Verfahren sind die den erfindungsgemässen Polyisocyanaten zugrundeliegenden Polyamine. Es handelt sich hierbei um gegebenenfalls Stellungs- und/oder Stereoisomerengemische darstellende Triamine der Formel (II)

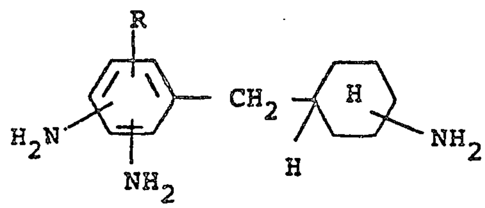
(II)

in welcher

R die bereits genannte Bedeutung hat.

Ausgangsmaterialien für die Herstellung der beim erfindungsgemässen Verfahren einzusetzenden Polyamine sind die entsprechenden aromatischen Triamine der Formel (III)

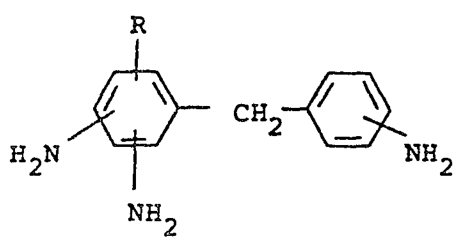
(III)

in welcher

R die bereits genannte Bedeutung hat.

Die Herstellung dieser aromatischen Triamine erfolgt in an sich bekannter Weise beispielsweise durch Umsetzung von N-[4(2)-Aminobenzyl]-anilin mit gegebenenfalls Alkyl-substituierten Phenylendiaminen gemäss Beilstein *13* H, Seite 309 bzw. gemäss DE-PS 107 718 oder durch Umsetzung (i) eines 3- oder 4-Nitrobenzylhalogenids, von Benzylalkohol oder eines Nitrobenzylchlorid-Isomerengemischs mit (ii) Nitrobenzol, alkylsubstituiertem Nitrobenzol, Alkylbenzol oder Benzol in Gegenwart eines Friedel-Crafts- oder ei-

nes Säurekatalysators, Nitrierung der so erhaltenen Umsetzungsprodukte zu den entsprechenden Trinitroverbindungen und Hydrierung der Nitrogruppen in Analogie zu EP-A-46917. Die nach der letztgenannten Methode erhaltenen Trinitroverbindungen und die hieraus hergestellten aromatischen Triamine stellen, herstelllungsbedingt, technische Gemische dar, die neben den entsprechenden trifunktionellen Verbindungen noch difunktionelle Verbindungen enthalten können. Aus diesen technischen Gemischen kann jedoch der geringe Anteil an difunktionellen Verbindung gewünschtenfalls auf der Aminstufe destillativ entfernt werden.

Typische Beispiele geeigneter Ausgangsmaterialien sind
2,4,4'(2')-Triaminodiphenylmethan,
2,6,4'(2')-Triaminodiphenylmethan,
4,6,4'(2')-Triamino-3-methyl-diphenylmethan,
2,6,4'(2')-Triamino-3-methyl-diphenylmethan,
3,5,4'(2')-Triamino-4-methyl-diphenylmethan,
2,6,4'(2')-Triamino-4-methyl-diphenylmethan,
3,5,4'(2')-Triamino-2-methyl-diphenylmethan,
4,6,4'(2')-Triamino-2-methyl-diphenylmethan,
oder deren Gemische. Auch die entsprechenden Ethyl-, Isopropyl-, n-Propyl- oder m-, iso- oder tert.-Butyl-substituierten Triamino-diphenylmethane können eingesetzt werden.

Bevorzugte Ausgangsmaterialien sind jedoch Isomerengemische von methylsubstituierten Triamino-diphenylmethanen oder deren technische Gemische mit den entsprechenden Diaminen, wie sie bei der Trinitrierung von 2- und/oder 4-Methyl-diphenylmethan bzw. von aus diesen Isomeren bestehenden Kohlenwasserstoffgemischen, Reduktion der Nitrogruppen und gegebenenfalls anschliessender destillativer Aufarbeitung anfallen. Die besonders bevorzugten, nach dieser Verfahrensweise hergestellten Ausgangsmaterialien bestehen im allgemeinen zu über 80 Gew.-% aus Triaminodiphenylmethanen, die ihrerseits zu über 90 Gew.-% aus Aminobenzyl-diaminotoluol-Isomeren bestehen.

Wie oben ausgeführt, kann es sich bei den Ausgangsmaterialien ausschliesslich um aromatische Triamine der Formel III bzw. und Gemische derartiger Triamine oder aber um Gemische derartiger Triamine mit den entsprechenden Diaminen handeln, welche vorzugsweise an jedem aromatischen Ring eine Aminogruppe aufweisen. In diesen Gemischen können bis zu 90, vorzugsweise bis zu 50 und insbesondere bis zu 20 Gew.-%, bezogen auf Gesamtgemisch, an solchen Diaminen vorliegen. Die herstellungsbedingt einen hohen Gehalt an derartigen aromatischen Diaminen aufweisenden Polyamingemische können gegebenenfalls vor der Herstellung der erfindungsgemäss einzusetzenden Ausgangsmaterialien durch partielle Hydrierung der aromatischen Polyamine ganz oder weitgehend destillativ von den Diaminen befreit werden. Es ist jedoch auch möglich, die aromatischen Polyamingemische als solche der Teilhydrierung zu unterziehen und gegebenenfalls die teilhydrierten erfindungsgemäss als Ausgangsmaterialien einzusetzenden Polyamingemische von den Diaminen und sonstigen, bei der Hydrierung anfallenden Nebenprodukten zu befreien. Schliesslich ist es ebenfalls möglich, nach der Durchführung des erfindungsgemässen Verfahrens eine destillative Abtrennung von difunktionellen Verfahrensprodukten (erhalten durch Phosgenierung der entsprechenden Diamine) und gegebenenfalls anderen Nebenprodukten durchzuführen, um zu weitgehend reinen Polyisocyanaten der Formel (I) zu gelangen.

Die Kernhydrierung der aromatischen Triamine erfolgt nach den bekannten Methoden des Standes der Technik, beispielsweise analog zu der in US-PS 2511028 beschriebenen Verfahrensweise. Hierbei werden die, gegebenenfalls im Gemisch mit den entsprechenden Diaminen vorliegenden, aromatischen Triamine unter Anlagerung von 3 Mol Wasserstoff pro Mol Ausgangsverbindung katalytisch hydriert, wobei unter «Ausgangsverbindung» gegebenenfalls das Gemisch der Triamine mit den Diaminen zu verstehen ist. Dies bedeutet, dass die Hydrierungsreaktion vorzugsweise nach Verbrauch von 3 Mol Wasserstoff pro Mol Ausgangsverbindung abgebrochen wird. Die Hydrierung erfolgt bei 20 bis 300° C, vorzugsweise bei 70 bis 200° C, insbesondere 120 bis 150° C unter einem Druck von 20 bis 300 bar, vorzugsweise 200 bis 300 bar.

Die Hydrierungsreaktion erfolgt in Gegenwart von 0,1 bis 20 Gew.-%, vorzugsweise von 0,1 bis 10 Gew.-%, bezogen auf katalytisch wirksames Metall einerseits und Triaminoverbindungen andererseits, eines Hydrierungskatalysators.

Geeignete Katalysatoren sind beispielsweise, gegebenenfalls auf inerten Trägern wie Aktivkohle, Kieselgel und insbesondere Aluminiumoxid, vorliegende Elemente der 8. Nebengruppe des Periodensystems der Elemente oder katalytisch wirksame organische Verbindungen dieser Elemente. Besonders gut geeignet sind zum Beispiel Ruthenium-, Platin-, Rhodium-, Nickel- und/oder Kobaltkatalysatoren in elementarer oder chemisch gebundener Form. Besonders bevorzugt werden Ruthenium oder katalytisch wirksame Rutheniumverbindungen eingesetzt. Beispiele geeigneter Rutheniumverbindungen sind Rutheniumdioxid, Rutheniumtetroxid, Bariumperruthenit, Natrium-, Kalium-, Silber-, Calcium- oder Magnesiumruthenat, Natriumperruthenat, Rutheniumpentafluorid, Rutheniumtetrafluoridhydrat oder Rutheniumtrichlorid. Falls Trägersubstanzen für die Katalysatoren mitverwendet werden, beträgt der Metallgehalt des Trägerkatalysators im allgemeinen 1 bis 10 Gew.-% vorzugsweise 1 bis 5 Gew.-%. Im übrigen ist selbstverständlich die Art und Menge des eingesetzten Katalysators keineswegs erfindungswesentlich, da die Hydrierungsreaktion nach den an sich bekannten Methoden des Standes der Technik erfolgt.

Es ist oft zweckmässig, die Hydrierungsreaktion in Gegenwart von Ammoniak durchzuführen, da Ammoniak unerwünschte Desaminierungsreaktionen und die Bildung von sekundären Aminen als Nebenprodukte unterdrückt. Falls Ammoniak mitverwendet wird, geschieht dies in Mengen von

0,1 bis 30 Gew.-%, vorzugsweise 5 bis 10 Gew.-%, bezogen auf zu hydrierende Ausgangsmaterialien.

Die Hydrierung kann lösungsmittelfrei oder in Gegenwart inerter Lösungsmittel durchgeführt werden. Im allgemeinen werden niedrigschmelzende bzw. flüssige Diamine in Substanz, hochschmelzende Diamine dagegen in gelöster Form hydriert. Als Lösungsmittel eignen sich unter den Reaktionsbedingungen inerte organische Verbindungen mit niedrigem Siedepunkt, vorzugsweise Alkohole wie Methanol, Ethanol, n-Propanol, i-Propanol, t-Butanol oder Ether, wie z.B. Dioxan, Tetrahydrofuran oder Diethylether oder Kohlenwasserstoffe wie Cyclohexan. Die Durchführung der Hydrierung findet z.B. kontinuierlich in einem Reaktionsrohr, einer Druckkesselkaskade oder vorzugsweise diskontinuierlich in einem Rührautoklaven statt, indem man den Autoklaven mit Katalysator, der zu hydrierenden Substanz und gegebenenfalls mit einem Lösungsmittel beschickt, mehrmals mit Inertgas spült und gegebenenfalls Ammoniak zudosiert. Danach drückt man Wasserstoff auf, bringt das Gemisch auf Reaktionstemperatur und hydriert, bis die theoretisch erforderliche Wasserstoffmenge absorbiert ist. Nach Abkühlen des Reaktionsgemisches und Abtrennung des Katalysators kann das Hydrierprodukt destillativ aufgearbeitet werden.

Die Hydrierungsprodukte fallen in hohen Ausbeuten an und lassen sich, erforderlichenfalls, destillativ von nicht umgesetzten aromatischen Aminen oder von den als Nebenprodukten entstehenden perhydrierten Di- und Triaminen, sowie den entsprechenden teilhydrierten Diaminen abtrennen. Auch nach einer derartigen destillativen Reindarstellung der beim erfindungsgemässen Verfahren einzusetzenden Triamine der Formel (II) stellen diese im allgemeinen Gemische von Stereo- und gegebenenfalls Stellungsisomeren dar. Durch die genannte destillative Aufarbeitung sind Triamine zugänglich, die zu über 80, vorzugsweise zu über 95 Gew.-% aus Polyaminen bestehen, die der allgemeinen Formel (II) entsprechen. Jedoch auch die nicht destillativ aufgearbeiteten Polyamine stellen erfindungsgemäss geeignete Ausgangsmaterialien dar, die jedoch nicht nur Stellungs- und/oder Stereoisomerengemische darstellen können, sondern darüber hinaus im Gemisch mit bis zu 90, vorzugsweise bis zu 50 und insbesondere bis zu 30 Gew.-% an anderen, gegebenenfalls alkylsubstituierten Di- und/oder Triaminen mit Diphenylmethan-, Benzyl-cyclohexan- oder Dicyclohexylmethan-Struktur vorliegen. Die genannten Diamine können dann vorliegen, wenn als Ausgangsmaterialien aromatische Triamine verwendet werden, die, wie oben ausgeführt, aromatische Diamine enthalten und/oder wenn es während der Hydrierungsreaktion im geringen Umfang zu einer Desaminierung des cycloaliphatischen Rings kommt. Eine derartige Desaminierung kann jedoch, wie oben ausgeführt, durch Mitverwendung von Ammoniak bei der Hydrierungsreaktion abgeschwächt werden.

Auf Grund NMR-spektroskopischer Untersuchungen wurde festgestellt, dass bei der Verwendung von Alkyl-substituierten aromatischen Triaminen als Ausgangsmaterial bei der Durchführung der Hydrierung die Alkylsubstituenten praktisch ausschliesslich am aromatischen Ring der Hydrierungsprodukte vorliegen.

Im allgemeinen ist eine destillative Reindarstellung der Hydrierungsprodukte vor ihrer Verwendung beim erfindungsgemässen Verfahren nicht erforderlich, da, wie bereits ausgeführt, gegebenenfalls eine destillative Aufarbeitung der erfindungsgemässen Verfahrensprodukte nach der Phosgenierungsreaktion durchgeführt werden kann. Ebensowenig kommt es bezüglich der Verwendbarkeit der Hydrierungsprodukte für das erfindungsgemässe Verfahren auf deren Stellungs- und Stereoisomerie bzw. auf die entsprechende Isomerenverteilung an.

Typische Beispiele von geeigneten Ausgangspolyaminen für das erfindungsgemässe Verfahren sind die den obengenannten aromatischen Triaminen entsprechenden aromatisch-cycloaliphatischen Triamine, die, wie gesagt, gewünschtenfalls von Nebenprodukten, wie sie bei der Hydrierungsreaktion oftmals entstehen, destillativ befreit werden können. Zu diesen Nebenprodukten gehören nicht nur die durch Desaminierung entstandenen Diamine mit Benzyl-cyclohexan-Struktur, sondern auch Di- und/oder Triamine mit Diphenylmethan- oder Dicyclohexylmethan-Struktur, die wegen der nicht streng selektiv verlaufenden Teilhydrierung im Gemisch mit den Triaminen der Formel (II) anfallen können. Von diesen Einschränkungen abgesehen, entsprechen die beim erfindungsgemässen Verfahren einzusetzenden Ausgangspolyamine den zu ihrer Herstellung eingesetzten, oben beispielhaft genannten aromatischen Polyaminen bzw. Polyamingemischen.

Bei der Durchführung des erfindungsgemässen Verfahrens zur Herstellung der neuen Triisocyanate erfolgt die Phosgenierung der Ausgangsamine oder deren Salze nach an sich bekannten Verfahren in Gegenwart eines inerten organischen Lösungsmittels (vgl. Houben-Weyl, Methoden der organischen Chemie, Georg Thieme Verlag Stuttgart (1952), Band 8, 4. Auflage, Seiten 120 ff.). Unter «Ausgangsaminen» sind in diesem Zusammenhang, den oben gemachten Ausführungen entsprechend, sowohl reine, gegebenenfalls Stellungs- und Stereoisomerengemische darstellende Verbindungen der allgemeinen Formel (II) als auch deren Gemische mit bis zu 90, vorzugsweise bis zu 50 und insbesondere bis zu 30 Gew.-% an anderen, gegebenenfalls Alkyl-substituierten Di- und/oder Triaminen mit Diphenylmethan-, Benzyl-cyclohexyl- oder Dicyclohexylmethan-Struktur zu verstehen, wobei sich diese Prozentangaben jeweils auf die Gesamtgemisch beziehen.

Als zu phosgenierende Salze eignen sich vorzugsweise Hydrochloride oder Ammoniumcarbamate, die durch Sättigung der Polyaminlösungen mit gasförmigem Chlorwasserstoff bzw. Kohlendioxid anfallen. Prinzipiell können auch andere Salze, die beispielsweise durch Neutralisation der Polyamine mit Protonen abspaltenden Säuren anfallen, phosgeniert werden.

Die Selektivität der Phosgenierungsreaktion ist weitgehend von der Aminkonzentration und vom Phosgenüberschuss abhängig. Vorzugsweise werden Phosgen in einem hohen molaren Überschuss und die zu phosgenierenden Amine in stark verdünnter Form eingesetzt. Im allgemeinen beträgt der molare Phosgenüberschuss 100 bis 2000%, vorzugsweise 100 bis 1000% und die Aminkonzentration, bezogen auf die Gesamtmenge an Amin einerseits und Lösungsmittel andererseits, 0,1 bis 15 Gew.-%, vorzugsweise 5 bis 10 Gew.-%.

Als Lösungsmittel können beliebige inerte organische Flüssigkeiten oder deren Gemische mit einem Siedepunkt von 60 bis 250° C, d.h. Halogenkohlenwasserstoffe, Aromaten, Hydroaromaten sowie deren Chlorverbindungen verwendet werden. Als Beispiele geeigneter Lösungsmittel seien Xylol, Mesitylen, Chlorbenzol, Dichlorbenzol, Trichlorbenzol, Chlornaphthalin und Dichlorethan genannt.

Die Reaktion wird entweder einstufig durch Heissphosgenierung bei Temperaturen von 100 bis 250° C oder zweistufig durch Kalt/Heissphosgenierung bei Temperaturen von −20 bis 250° C unter Normaldruck durchgeführt.

Bei Verwendung der freien Amine als Ausgangsverbindung (Basenphosgenierung) wird zuerst bei Temperaturen von −20 bis +60° C Ammoniumcarbamidsäurechlorid hergestellt, das dann bei Temperaturen von 20 bis 250° C mit Phosgen zum Polyisocyanat weiterreagiert.

Die Reinigung der Verfahrensprodukte erfolgt im allgemeinen nach Entphosgenierung durch Abdampfen des Lösungsmittels und anschliessende Destillation unter vermindertem Druck.

Die erfindungsgemässen Verfahrensprodukte, d.h. die neuen erfindungsgemässen Triisocyanate fallen in hohen Ausbeuten als farblose bis gelb gefärbte, niedrigviskose Flüssigkeiten an und stellen wertvolle Aufbaukomponenten bei der Herstellung von Polyurethankunststoffen nach dem Isocyanat-Polyadditionsverfahren dar. Die Stellungs- und/oder Stereoisomerie der neuen Triisocyanate entspricht weitgehend der Isomerie der bei der Phosgenierung eingesetzten Triamine. Im allgemeinen ist es nicht erforderlich, die beim erfindungsgemässen Verfahren anfallenden Gemische in einzelne Stellungs- und/oder Stereoisomere aufzutrennen, da die erfindungsgemässen Verfahrensprodukte ohne derartige Trennungsoperationen direkt der erfindungsgemässen Verwendung zugeführt werden können. Wie bereits ausgeführt können gegebenenfalls im Gemisch mit den erfindungsgemässen Triisocyanaten vorliegende Diisocyanate und sonstige Nebenprodukte (insbesondere Phosgenierungsprodukte von Triaminen mit Diphenylmethan- oder Dicyclohexylmethan-Struktur) destillativ von den erfindungsgemässen Triaminen ganz oder teilweise abgetrennt werden. Für viele Anwendungsgebiete der erfindungsgemässen Triisocyanate ist jedoch eine derartige Reindarstellung nicht erforderlich, da auch die entsprechenden Polyisocyanatgemische, die beispielsweise mindestens 50 Gew.-%, vorzugsweise mindestens 80 Gew.-% an erfindungsgemässen Polyisocyanaten der Formel (I) aufweisen, wertvolle neue Ausgangsmaterialien für die Polyurethanchemie darstellen. Die neuen erfindungsgemässen Triisocyanate bzw. ihre Gemische mit den genannten Nebenprodukten, welche mindestens 50 Gew.-%, vorzugsweise mindestens 80 Gew.-% an erfindungsgemässen Triisocyanaten der Formel (I) enthalten, können besonders vorteilhaft zur Herstellung von Polyurethanlacken bzw. Beschichtungsmaterialien eingesetzt werden, wobei sie bei den an sich bekannten Verfahren zur Herstellung derartiger Kunststoffe anstelle oder zusammen mit den bisher eingesetzten Polyisocyanaten zum Einsatz gelangen. Besonders vorteilhaft werden die neuen erfindungsgemässen Triisocyanate bzw. Polyisocyanatgemische bei der Herstellung von Polyurethankunststoffen der beispielhaft genannten Art nach dem Präpolymerprinzip verwendet.

Die nachfolgenden Beispiele dienen der weiteren Erläuterung der Erfindung. Alle Prozentangaben beziehen sich, soweit nicht Anderslautendes vermerkt, auf Gewichtsprozente. Die Analyse der Isomerenverteilung der Zwischen- und Endprodukte erfolgte gaschromatographisch.

*Beispiel 1*

a) In einem 700 ml Rührautoklaven wurden 350 g eines Amingemisches, bestehend aus 1,4% 4,4'-Diamino-diphenylmethan, 10,2% 4,6,2'-Triamino-3-methyl-diphenylmethan, 6,8% 2,6,4'-Triamino-3-methyl-diphenylmethan, 78,7% 4,6,4'-Triamino-3-methyl-diphenylmethan und 2,9% an anderen aromatischen Polyaminen und 35 g Ruthenium-Aluminiumoxid-Trägerkatalysator (5% Ru) vorgelegt. Nach mehrmaligem Spülen mit Stickstoff wurden 35 g flüssigen Ammoniaks zugepumpt und das Gemisch bei 140° C und 275 bar hydriert, bis nach einer Reaktionszeit von 14,7 Stunden die theoretisch für die angestrebte Teilhydrierung erforderliche Wasserstoffmenge verbraucht war. Der Autoklav wurde nun auf 70° C abgekühlt, entspannt und das Produkt in 350 ml Methanol aufgenommen. Der Katalysator wurde abfiltriert, mit Methanol gewaschen und die vereinigten Filtrate destilliert. Bei einer Siedetemperatur von 110 bis 220° C/0,4 bis 1 mbar gingen 299,5 g Rohprodukt über, das folgende Zusammensetzung besass:

3,4% Diamino-methyldicyclohexylmethan (Isomerengemisch)

4,4% Diamino-methylbenzylcyclohexan (Isomerengemisch)

4,3% Triamino-methyldicyclohexylmethan (Isomerengemisch)

82,8% Diamino-methylbenzyl-cyclohexylamin (Isomerengemisch)

1,8% Triamino-methyl-diphenylmethan (Isomerengemisch)

3,3% unbekannte Polyamine

b) Zu einer Lösung von 600 g Phosgen in 2 l Chlorbenzol wurden bei 0° C 280 g des Rohpro-

dukts aus Beispiel 1a) gelöst in 2 l Chlorbenzol unter Rühren und Kühlen so zugetropft, dass die Reaktionstemperatur von 0° C nicht überschritten wurde.

Die entstandene Suspension wurde unter Einleiten von 300 g/h Phosgen bis zum Rückfluss erhitzt und anschliessend weitere 4 Stunden unter gleichen Bedingungen gekocht. Dabei entstand eine klare Lösung, die noch weitere 2 Stunden unter Rückfluss mit Phosgen begast wurde. Die Lösung wurde durch Einleiten von Stickstoff entphosgeniert, das Lösungsmittel unter vermindertem Druck abdestilliert und das Produkt bei 100 bis 220° C/0,5 bis 0,8 mbar destilliert.

Nach erneuter Destillation wurden 222 g einer bei 155 bis 170° C/0,1 mbar siedenden Flüssigkeit gewonnen, die laut gaschromatographischmassenspektroskopischer Untersuchung folgende Zusammensetzung aufwies:

3,4% Diisocyanato-methylbenzylcyclohexan (Isomerengemisch)
81,5% Diisocyanato-methylbenzylcyclohexylisocyanat (Isomerengemisch)
4,4% Triisocyanato-methyl-diphenylmethan (Isomerengemisch)
10,7% unbekannte Polyisocyanate

Das Produkt besass einen NCO-Gehalt von 38,7% und eine Viskosität von 180 mPa·sec/25° C.

*Beispiel 2*

175 g eines Gemisches aus 97,9% Diamino-methylbenzyl-cyclohexylamin (Isomerengemisch) und 2,1% Triamino-methyldiphenylmethan (Isomerengemisch), das durch Nitrierung eines Methyl-diphenylmethan-Isomerengemisches, katalytische Hydrierung der Nitrogruppen, kernhydrierung analog Beispiel 1a) und anschliessende destillative Aufarbeitung hergestellt worden war, wurden in 2 l wasserfreiem Chlorbenzol gelöst. Die Aminlösung wurde langsam unter Rühren bei −10° C in eine Lösung von 450 g Phosgen in 2 l Chlorbenzol getropft. Dabei bildete sich eine schwach gefärbte Suspension, die unter Einleiten von 300 g/h Phosgen langsam erwärmt wurde. Bei einer Reaktionstemperatur von 60° C kam es zu einer heftigen Chlorwasserstoffentwicklung, die Suspension färbte sich orange und ging bei 125° C in eine klare Lösung über. Nach 1,5-stündigem Kochen wurde der Phosgenstrom abgestellt und statt dessen im Verlauf einer Stunde Stickstoff eingeleitet. Das Lösungsmittel wurde abgedampft und nach Flashdestillation bei 180 bis 200° C/0,2 bis 0,3 mbar 190 g Rohisocyanat erhalten. Nach erneuter Destillation unter vermindertem Druck wurden 185 g Diisocyanato-methylbenzyl-cyclohexylisocyanat (Isomerengemisch), das, bezogen auf Gesamtgemisch, 4,9% Diisocyanate mit Benzyl-cyclohexan-Struktur enthielt und durch folgende Daten charakterisiert war:

| | |
|---|---|
| NCO-Gehalt: | 39,8% |
| Gehalt an hydrolysierbarem Chlor: | 0,04% |
| Siedepunkt: | 158 bis 162° C/0,05 mbar |
| Viskosität: | 203 mPas/25° C |

*Beispiel 3*

a) In einem 1,3 l Rührautoklaven wurden 380 g eines Gemisches bestehend aus

0,4% 2,6,4′-Triaminodiphenylmethan
9,8% 2,4,2′-Triaminodiphenylmethan
89,8% 2,4,4′-Triaminodiphenylmethan

380 ml tert.-Butanol und 38 g Ruthenium (5%)-$Al_2O_3$-Trägerkatalysator vorgelegt. Nach mehrmaligem Spülen mit Stickstoff und Wasserstoff wurden 100 bar Wasserstoff aufgedrückt und das Gemisch auf 140° C erhitzt. Unter intensivem Rühren wurde nun bei einem Druck von 275 bar hydriert, bis nach 8,5 Stunden die theoretisch für die angestrebte Teilhydrierung erforderliche Wasserstoffmenge verbraucht war. Der Autoklav wurde auf 60° C abgekühlt, entspannt, der Katalysator abgesaugt und das Produkt einer Flashdestillation unterworfen. Dabei wurden 285 g eines bei 155 bis 220° C/0,6 mbar siedenden Amingemisches gewonnen, das zu 10,1% aus Diamino-dicyclohexylmethan, 29,0% aus Triamino-dicyclohexylmethan, 51,6% aus Diaminobenzyl-cyclohexylamin und 9,3% aus anderen nicht identifizierten Aminen bestand. Nach Feindestillation bei 210° C/0,5 mbar wurden 136 g Diaminobenzyl-cyclohexylamin (Isomerengemisch) isoliert, das, bezogen auf Gesamtgemisch, 6,2% Triamino-dicyclohexylamin (Isomerengemisch) enthielt.

b) 136 g Amingemisch aus Beispiel 3a) wurden in 1 l wasserfreiem Chlorbenzol gelöst. Dann wurde Kohlendioxid bis zur Sättigung eingeleitet und die dabei entstandene Suspension bei −10° C unter intensivem Rühren in eine Lösung von 400 g Phosgen in 1 l Chlorbenzol getropft. Das Gemisch wurde mit 15 l/h Phosgen begast und im Verlauf 75 Minuten zum Rückfluss erhitzt. Nach 3,5-stündigem Rühren löste sich der Feststoff vollständig auf. Die Lösung wurde weitere 2 Stunden phosgeniert, anschliessend überschüssiges Phosgen mit Stickstoff ausgeblasen und Chlorbenzol abdestilliert. Das Rohprodukt wurde durch Flashdestillation bei einem Vakuum von 1 mbar von polymeren Nebenprodukten befreit und anschliessend erneut destilliert. Dabei wurden 145 g Isocyanat gewonnen, das einen Siedepunkt von 182 bis 185° C/0,6 mbar aufwies und zu 91,7% (76,8% der Theorie) aus Diisocyanatobenzylcyclohexylisocyanat-Isomeren, 2,2% Triisocyanatodicyclohexylmethan-Isomeren und 6,1% unbekannten Isocyanaten bestand. Der NCO-Gehalt betrug 41,8%, der Gehalt an hydrolisierbarem Chlor 600 ppm und die Viskosität 98 mPa·s/25° C.

*Beispiel 4*

a) In einem 1,3 l Rührautoklaven wurden 253 g 2,4,4′-Triaminodiphenylmethan, 253 g tert.-Butanol und 25,3 g Ru/$Al_2O_3$-Katalysator vorgelegt und das Gemisch unter intensivem Rühren bei 140° C/275 bar hydriert. Nach einer Reaktionszeit von 10 Stunden war die theoretisch erforderliche Wasserstoffaufnahme beendet. Der Autoklav wurde auf 60° C abgekühlt und entleert. Danach wurde der Katalysator abfiltriert, gewaschen und das

Rohprodukt destilliert. Im Siedebereich von 110 bis 184° C/0,5 mbar destillierten 234 g eines Amingemisches über das zu 61,9% aus 4-(2,4-Diaminobenzyl)-cyclohexylamin, 30,7% aus 2,4,4'-Triamino-dicyclohexylmethan, 3,5% aus verschiedenen Diamino-dicyclohexylmethanen und 3,9% aus unbekannten Aminen bestand. Nach Feindestillation bei 184° C/0,5 mbar wurden 137 g 4-(2,4-Diaminobenzyl)-cyclohexylamin isoliert, das, bezogen auf Gesamtgemisch, 4,4% 2,4,4'-Triamino-dicyclohexylmethan enthielt.

b) Durch Einleiten von Kohlendioxid in eine Lösung von 136 g des destillierten Polyamingemischs aus Beispiel 4a) in 1 l Chlorbenzol wurde eine Ammoniumcarbamat-Suspension hergestellt. Diese Suspension wurde bei −10° C unter Rühren zu einer Lösung von 400 g Phosgen in 1 l Chlorbenzol gegeben, 15 Minuten gerührt und der Ansatz unter Einleiten von 15 l Phosgen im Verlauf einer Stunde zum Rückfluss erhitzt. Nach 2-stündiger Heissphosgenierung war der Feststoff vollständig aufgelöst. Die Mischung wurde noch weitere 4 Stunden phosgeniert und durch Einleiten von Stickstoff entphosgeniert. Nun wurde das Lösungsmittel abdestilliert, das Rohprodukt durch Flashdestillation vorgereinigt und bei 185° C/0,6 mbar erneut destilliert. Dabei konnten 150 g Isocyanat isoliert werden, das folgende Zusammensetzung besass:

96,2% 4-(2,4-Diisocyanatobenzyl)-cyclohexylisocyanat
2,8% 2,4,4'-Triisocyanato-dicyclohexylmethan
1,0% unbekannte Polyisocyanate

Das Produkt war durch folgende Daten charakterisiert:

| | |
|---|---|
| NCO-Wert: | 42,0% |
| Gehalt an hydrolisierbarem Chlor: | 30 ppm |
| Viskosität/25° C: | 98 mPa·s |

**Patentansprüche**

1. Gegebenenfalls Stellungs- und/oder Stereoisomerengemische darstellende Triisocyanate der Formel (I)

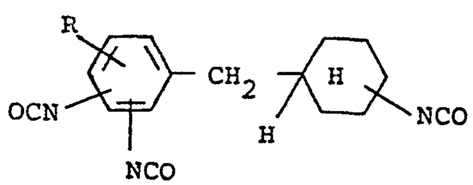

(I)

in welcher

R für Wasserstoff oder einen verzweigten oder linearen Alkylrest mit 1 bis 4 Kohlenstoffatomen steht.

2. Verfahren zur Herstellung von Triisocyanaten gemäss Anspruch 1, dadurch gekennzeichnet, dass man die ihnen zugrundeliegenden araliphatischen Polyamine in an sich bekannter Weise phosgeniert.

3. Verwendung der Triisocyanate gemäss Anspruch 1 als Aufbaukomponente für Polyurethankunststoffe nach dem Isocyanat-Polyadditionsverfahren.

**Claims**

1. Triisocyanates, optionally in the form of position and/or stereoisomeric mixtures, of the formula (I)

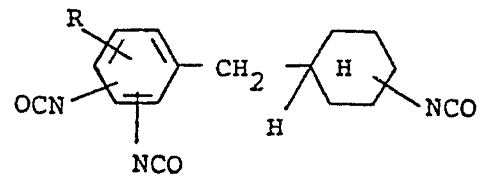

(I)

in which

R represents hydrogen or a branched or linear alkyl group having 1 to 4 carbon atoms.

2. Process for the preparation of triisocyanates according to claim 1, characterised in that the araliphatic polyamines on which they are based are phosgenated in known manner.

3. Use of the triisocyanates according to claim 1 as components for the synthesis of polyurethane plastics by the isocyanate polyaddition process.

**Revendications**

1. Triisocyanates représentant éventuellement des mélanges d'isomères de position et/ou de stéréo-isomères, de formule (I)

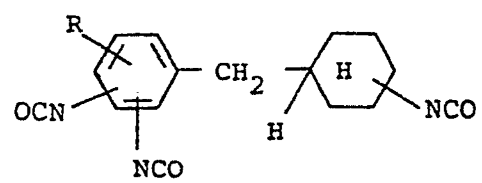

(I)

dans laquelle

R représente l'hydrogène ou un reste alkyle ramifié ou linéaire avec 1 à 4 atomes de carbone.

2. Procédé de préparation de triisocyanates selon la revendication 1, caractérisé en ce que l'on phosgène de façon connue en soi leurs polyamines araliphatiques constitutives.

3. Utilisation des triisocyanates selon la revendication 1 comme éléments structuraux pour des matières synthétiques à base de polyuréthannes selon le procédé de polyaddition d'isocyanates.